# EUROPEAN PATENT SPECIFICATION

(11) **EP 0 903 116 B1**
(45) Date of publication and mention of the grant of the patent: **16.07.2003**
(21) Application number: 98111957.1
(22) Date of filing: 29.06.1998
(51) Int. Cl.: A61C 8/00, A61B 17/16

(54) **An assembly of a mill-reamer and a prosthesis for bone implantology by a screwed pin**
Fräswerkzeug und Prothese für die Knochenimplantologie mittels eines mit einem Gewinde versehenen Stiftes
Ensemble de fraise et prothèse pour l'implantologie osseuse au moyen d'un pivot fileté

(30) Priority: 18.09.1997 IT MI972117
(43) Date of publication of application: 24.03.1999
(73) Proprietor: Veneziano, Walter, 21022 Azzate (VA) (IT)
(72) Inventor: Veneziano, Walter, 21022 Azzate (VA) (IT)
(74) Representative: Ferraiolo, Ruggero

(56) References cited:
- EP-A- 0 557 899
- WO-A-94/20247
- GB-A- 1 419 273
- US-A- 4 706 659
- US-A- 5 536 268

## Description

The present invention concerns an assembly of a mill-reamer and a prosthesis for bone implantology by a screwed pin, especially to be used in dentistry, orthopaedics, and facial-maxillary surgery, the prosthesis being suited to being screwed into the bone seat as produced by the mill-reamer and receiving a prosthesis connection element.

The field in which the invention is applied is that of the anchorage of said prosthesis exploiting the osteo-integration phenomenon, that is the natural response of the bone tissue when it re-generates and strengthens around the walls of the prosthesis implanted in the bone-seat.

The prior art comprises various forms of prosthesis, each conceived according to a certain "school". The more advanced in the field are the Swiss, Swedish, German, American and Italian schools. These prostheses differ in that their external shape is generally cylindrical or frustum shaped, with or without thread, while application instruments and techniques may be similar or identical.

The applicant has the professional experience to acknowledge the various techniques and diverse instruments and that they do not disclose the contents of the claims in the present patent application; especially, he knows of the Italian patent n° 1237496 that discloses a mill defined by the inventor as "upturned wedding cake" characterized by a series of cutting sections that are cylindrical, concentric and with decreasing diameter along the mill; the latter prepares a seat for a prosthesis that is sensibly frustum shaped bearing two threads, a first one fast-step suited to screwing into the trabecular bone-tissue, and a second, possibly self-threading, to be screwed in the cortical part opposite that where the prosthesis is inserted, this second thread bears a number of single threads that is a multiple of those on the former.

The drawbacks of the prior art mainly arise from the fact that they involve rather long and traumatic operations because they employ a high number of instruments (mills, tapping machines, reamers and prostheses), moreover, geometric differences, especially between the bone-seat and the prosthesis, produce slow bone healing, called "second thought", known to feature scarce reliability and duration.

The present invention obviates the above drawbacks and, as characterized in the claims, is an assembly of a mill-reamer and a prosthesis for bone implantology by a screwed pin, wherein the mill-reamer has a sensibly cylindrical-conical shape and comprises at least two ribbings that are sensibly longitudinal and of equal angular distance from one another, each ribbing bearing cutting conical parts longitudinally alternated with non cutting cylindrical parts, longitudinal slots being formed between adjacent ribbings, the prosthesis having a conical-cylindrical shape in which the conical part comprises a helical thread .

The main advantages of the invention are that: the mill-reamer, with its conical part, produces the desired conical seat in a progressive, accurate and non traumatic way; the longitudinal slots allow an effective out-flow of cooling and physiological liquids along with bone debris; the prosthesis is easily engaged in the bone seat in an accurate conical coupling without producing compression of the seat walls and without concentrating the stress that cause the known bone absorption and the consequent loss of implant integration.

The invention is described in detail below with an example of embodiment and the support of drawings in which:
Fig. 1 is a view of a first mill-reamer,
Fig. 2 is a partly sectioned view of a prosthesis, and
Fig. 3 is a view of a second mill-reamer.

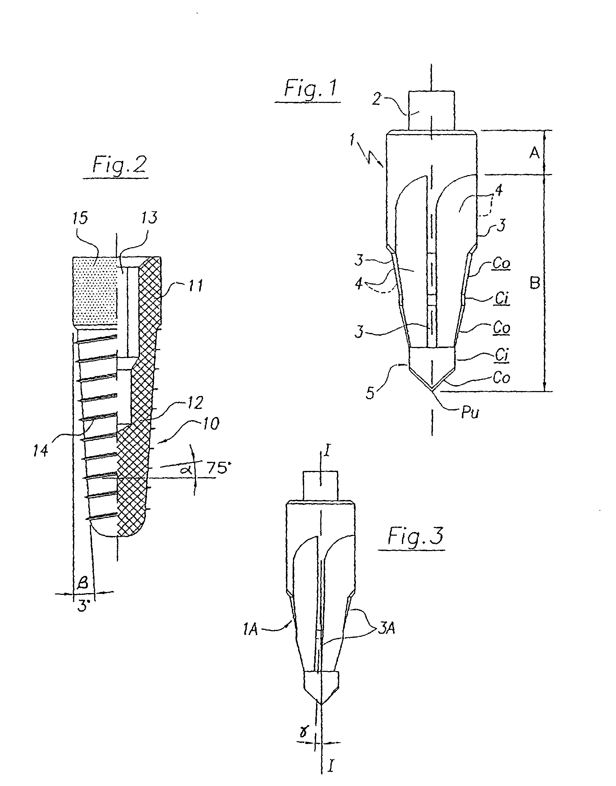

Fig. 1 shows a mill-reamer 1 that bears a shank 2 at the top onto which the clamp of the micro-motor, or handle (not shown), that drives the instrument is tightened; the body of the mill-reamer comprises two parts, an upper part A that is completely cylindrical, and a lower part B in which there are four longitudinal ribbings 3, four longitudinal slots 4, and a conical-cylindrical tip 5. Each longitudinal ribbing 3 and the tip 5 are cutting in the conical parts Co and not cutting in the cylindrical parts Ci ; the tip Pu of the conical-cylindrical tip 5 is, obviously, used to accurately position the instrument in the operation field and the cylindrical part Ci is used to correctly pilot the instrument in the initial stage of its work, and this makes it easier to proceed in an aligned production of the bone seat.

Fig. 2 shows prosthesis 10 that bears the cylindrical part 11 at the top and the conical part 12 at the bottom; a longitudinal cavity 13 is opened at the top of the cylindrical part 11 and extends in part also within the conical part 12 in order to receive the foreseen prosthesis connection element, which element may be, for instance, a dental prosthesis (not shown) cemented therein; a helical thread 14 is formed on the wall of the conical part 12. The angle α of this thread, equal to 75° in the example, and the angle β of the conical part 12 (equal to 3° in the example), as also the pitch of the thread and the out-crop of the threads on the wall of the conical part, will have, for each prosthesis, values such as to confer the prosthesis in the best working and resistance conditions according to the bone architecture present in the operation field. The lateral part of the cylindrical part 11 bears a sort of embossing or tapping 15, in relief or recesses, with the purpose of increasing the contact surface and sealing of the prosthesis in the same bone.

Fig. 3 shows a mill-reamer 1A the same as that shown in Fig. 1, but in which the longitudinal ribbings 3A, have an angle γ with the I-I axis of the instrument.

It is understood that the number of ribbings in the mill-reamers is adequate for the bone architecture featured by each case.

## Claims

1. An assembly of a mill-reamer (1) and a prosthesis (10) for bone implantology by a screwed pin, the prosthesis comprising a cylindrical part (11) at the top with a central seat (13) to receive a prosthesis connecting element, wherein the mill-reamer (1) has a sensibly cylindrical-conical shape comprising at least two ribbings (3) that are sensibly longitudinal and of equal angular distance from one another, each ribbing bearing cutting conical parts (Co) longitudinally alternated with non cutting cylindrical parts (Ci), longitudinal slots (4) being formed between adjacent ribbings and the prosthesis (10) has a conical-cylindrical shape in which the conical part comprises a helical thread (14).

2. An assembly according to claim 1 **characterized in that** the sensibly longitudinal ribbings (3) have an angle (γ) with the longitudinal axis (I-I) of the mill-reamer (1A).

3. An assembly according to claim 1 **characterized in that** the mill-reamer (1) comprises a lower end with a conical-cylindrical tip (5) bearing a non cutting cylindrical part (Ci) and a cutting conical part (Co).

4. An assembly according to claim 1 **characterized in that** the lateral surface of the cylindrical part (11) of the prosthesis (10) reliefs or recesses (15) are formed so as to increase the surface contract with the bone and the sealing of the prosthesis within the bone.

## Patentansprüche

1. Ensemble aus einem Fräswerkzeug (1) und einer Prothese (10) für Knochenimplantologie mittels eines Gewindestifts, wobei die Prothese einen zylindrischen oberen Teil (11) mit einem mittigen Sitz (13) zur Aufnahme eines Prothesen-Anschlusselements aufweist, wobei das Fräswerkzeug (1) eine im wesentlichen zylindrisch-konische Form hat mit mindestens zwei Rippen (3), die im wesentlichen längs verlaufen und gleichen Winkelabstand voneinander haben, wobei jede Rippe konische schneidende Teile (Co) aufweist, die in Längsrichtung mit zylindrischen nichtschneidenden Teilen (Ci) abwechseln, wobei Längsschlitze (4) zwischen benachbarten Rippen gebildet sind und die Prothese (10) eine konisch-zylindrische Form hat, bei der der konische Teil einen Gewindegang (14) aufweist.

2. Ensemble nach Anspruch 1,
**dadurch gekennzeichnet, dass** die im wesentlichen längsverlaufenden Rippen (3) einen Winkel (γ) mit der Längsachse (I-I) des Fräswerkzeugs (1A) einschliessen.

3. Ensemble nach Anspruch 1,
**dadurch gekennzeichnet, dass** das Fräswerkzeug (1) ein unteres Ende mit einer konisch-zylindrischen Spitze (5) aufweist, die einen zylindrischen nichtschneidenden Teil (Ci) und einen konischen schneidenden Teil (Co) trägt.

4. Ensemble nach Anspruch 1,
**dadurch gekennzeichnet, dass** in der Seitenfläche des zylindrischen Teils (11) der Prothese (10) Erhöhungen oder Vertiefungen (15) gebildet sind, derart, dass der Oberflächenkontakt mit dem Knochen und die Abdichtung der Prothese innerhalb des Knochens verstärkt wird.

## Revendications

1. Ensemble d'une fraise-alésoir (1) et d'une prothèse (10) destinée à être implantée dans un os, la prothèse comprenant dans sa partie haute une partie cylindrique (11) munie d'un logement central (13) destiné à recevoir un élément de raccordement de prothèse,
dans lequel
la fraise- alésoir (1) a une forme sensiblement cylindro-conique qui comprend au moins deux nervures (3) qui sont sensiblement longitudinales et à égale distance angulaire l'une de l'autre, chaque nervure portant des parties coniques coupantes (Co) qui alternent longitudinalement avec des parties cylindriques non coupantes (Ci), des rainures longitudinales (4) étant formées entre les nervures adjacentes, et la prothèse (10) a une forme conico-cylindrique dans laquelle la partie conique comprend un filet hélicoïdal (14).

2. Ensemble selon la revendication 1,
**caractérisé en ce que**
les nervures sensiblement longitudinales (3) forment un angle (γ) avec l'axe longitudinal (I-I) de la fraise-alésoir (1A).

3. Ensemble selon la revendication 1,
**caractérisé en ce que**
la fraise-alésoir (1) comprend une extrémité inférieure munie d'une pointe conico-cylindrique (5) qui porte une partie cylindrique non coupante (Ci) et une partie conique coupante (Co).

4. Ensemble selon la revendication 1,
**caractérisé en ce que**
des reliefs ou des creux (15) sont formés dans la surface latérale de la partie cylindrique (11) de la prothèse (10) pour agrandir la surface de contact avec l'os et le scellement de la prothèse dans l'os.
